(19) Europäisches Patentamt
European Patent Office
Office européen des brevets

(11) **EP 4 290 530 A1**

(12) **EUROPÄISCHE PATENTANMELDUNG**

(43) Veröffentlichungstag:
**13.12.2023 Patentblatt 2023/50**

(21) Anmeldenummer: **23176088.5**

(22) Anmeldetag: **30.05.2023**

(51) Internationale Patentklassifikation (IPC):
**G16H 50/20** (2018.01)       **G16H 50/30** (2018.01)

(52) Gemeinsame Patentklassifikation (CPC):
**G16H 50/20; G16H 50/30**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **07.06.2022 DE 102022114248**

(71) Anmelder: **TCC GmbH**
**22083 Hamburg (DE)**

(72) Erfinder:
• **Krannich, Alexander**
**14641 Nauen (DE)**
• **Storm, Christian**
**22359 Hamburg (DE)**
• **Barg, David**
**22179 Hamburg (DE)**

(74) Vertreter: **Michalski Hüttermann & Partner**
**Patentanwälte mbB**
**Kaistraße 16A**
**40221 Düsseldorf (DE)**

(54) **VERFAHREN SOWIE VORHERSAGESYSTEM ZUR ERMITTLUNG DER EINTRITTSWAHRSCHEINLICHKEIT EINER SEPSIS EINES PATIENTEN**

(57)     Die Erfindung betrifft ein Verfahren sowie ein Vorhersagesystem zur Ermittlung der Eintrittswahrscheinlichkeit einer Sepsis eines Patienten. Erfindungsgemäß vorgesehen ist dazu ein sukzessives Erfassen von jeweiligen Werten (33, 34, 35, 36) für wenigstens vier vordefinierte gesundheitsspezifische Parameter des Patienten (37) über einen vorbestimmten Erfassungszeitraum (32), das Ermitteln eines Eingabewertes (24, 25, 26, 27) für jeden gesundheitsspezifischen Parameter, wobei dieser Eingabewert (24, 25, 26, 27) abhängig ist von einem Wert größer oder gleich dem 0,45-Quantil und kleiner oder gleich dem 0,55 Quantil der über den vorbestimmten Erfassungszeitraum (32) sukzessive für den jeweiligen gesundheitsspezifischen Parameter erfassten Werte (33, 34, 35, 36), das Eingeben der Eingabewerte (24, 25, 26, 27) für die vier vordefinierten gesundheitsspezifischen Parameter in ein auf einem Computer (6) implementiertes Regressionsmodell oder künstliches neuronales Netzwerk (20), wobei das Regressionsmodell bzw. das künstliche neuronale Netzwerk (20) auf die Eingabe der Eingabewerte (24, 25, 26, 27) eine Eintrittswahrscheinlichkeit liefert, mit der bei dem Patienten nach einer vorbestimmten Dauer eine Sepsis auftritt (28). Damit wird es ermöglicht, die Genauigkeit der Angabe der Eintrittswahrscheinlichkeit einer Sepsis zu verbessern.

Fig. 1

**Beschreibung**

Die Erfindung betrifft ein Verfahren zur Ermittlung der Eintrittswahrscheinlichkeit einer Sepsis eines Patienten mit folgenden Verfahrensschritten: Erfassen von jeweiligen Werten für wenigstens vier vordefinierte gesundheitsspezifische Parameter und Eingabe der Werte für die vordefinierten gesundheitsspezifischen Parameter in ein auf einem Computer implementiertes Regressionsmodell oder künstliches neuronales Netzwerk, wobei das Regressionsmodell bzw. das künstliche neuronale Netzwerk auf die Eingabe der Eingabewerte eine Eintrittswahrscheinlichkeit liefert, mit der bei dem Patienten nach einer vorbestimmten Dauer eine Sepsis auftritt.

Eine Sepsis ist definiert als ein lebensbedrohlicher Zustand, der entsteht, wenn die körpereigenen Abwehrreaktionen gegen eine Infektion die eigenen Gewebe und Organe schädigen. Sie ist eine der schwersten Komplikationen von Infektionskrankheiten, die durch Bakterien, Viren, Pilze oder Parasiten ausgelöst werden. Für die Ermittlung der Eintrittswahrscheinlichkeit einer Sepsis ist es bekannt, gesundheitsspezifische Parameter zu messen und an ein Regressionsmodell oder ein künstliches neuronales Netzwerk zu übergeben. Das Regressionsmodell bzw. das künstliche neuronale Netzwerk bestimmt mithilfe dieser Werte dann die Wahrscheinlichkeit des Eintretens einer Sepsis für den Patienten. So beschreibt die WO 2017/165693 A1 ein System zur Krankheitsvorhersage umfassend eine Verarbeitungsschaltung, die so konfiguriert ist, dass sie einen Datensatz empfängt, der Daten einer Patientenpopulation enthält, wobei die Daten für jeden aus einer Vielzahl von Patienten der Patientenpopulation Werte für eine Vielzahl von Merkmalen und einen Diagnosewert enthalten, der anzeigt, ob eine Krankheit diagnostiziert wurde. Die Verarbeitungsschaltung ist so konfiguriert, dass sie auf der Grundlage von Korrelationen zwischen den Werten aus dem Datensatz eine Vielzahl von Teilmengen der Merkmale auswählt und für jede von mindestens einer der Teilmengen einen maschinellen Lernprozess mit der jeweiligen Teilmenge und den Diagnosewerten als Eingabeparameter ausführt, wobei die Ausführung ein jeweiliges Vorhersagemodell erzeugt. Die Verarbeitungsschaltung ist so konfiguriert, dass sie das jeweilige Vorhersagemodell ausgibt.

Die DE10 2020 214 050 A1 beschreibt ein computerimplementiertes Verfahren zum Bereitstellen von mindestens einem Prognosewert für mindestens eine medizinische Laborgröße, insbesondere zur Anwendung in einer medizinischen Laborwertanalyse, mit folgenden Schritten:- Bereitstellen von mindestens einem Laborwertverlauf, der einen Verlauf von historischen Laborwerten der mindestens einen Laborgröße zu mindestens zwei historischen Zeitpunkten angibt;- Ermitteln mindestens eines Laborgrößenmerkmals für jede der mindestens einen Laborgröße aus dem entsprechenden Laborwertverlauf;- Bestimmen des mindestens einen Prognosewerts zu einem vorgegebenen Prognosezeitpunkt abhängig von einem trainierten, datenbasierten Prognosemodell und abhängig von dem mindestens einen Laborgrößenmerkmal für jeden des mindestens einen Laborwertverlaufs.

Die WO 2006/ 061 644 A1 beschreibt ein System und eine Methode zur Erkennung früher Anzeichen einer Infektion und insbesondere zur Identifizierung von Personen, bei denen eine Sepsis am wahrscheinlichsten ist. Die Messung des Expressionsniveaus bestimmter Kombinationen von Zytokinen und/oder zellulären Aktivierungsmarkern, gegebenenfalls in Kombination mit der Verwendung von Vorhersagealgorithmen, ermöglicht eine hohe Vorhersagegenauigkeit. Die Methode ist sowohl im zivilen als auch im militärischen Bereich anwendbar.

Aus der Praxis bekannte System liefern dabei bisher aber nur eine geringe Verlässlichkeit der Vorhersage.

Davon ausgehend ist es die Aufgabe der Erfindung, die Genauigkeit der Angabe der Eintrittswahrscheinlichkeit einer Sepsis zu verbessern.

Diese Aufgabe wird durch die Gegenstände der unabhängigen Ansprüche gelöst. Bevorzugte Weiterbildungen finden sich in den Unteransprüchen.

Erfindungsgemäß ist also ein Verfahren vorgesehen zur Ermittlung der Eintrittswahrscheinlichkeit einer Sepsis eines Patienten mit folgenden Verfahrensschritten: sukzessives Erfassen von jeweiligen Werten für wenigstens vier vordefinierte gesundheitsspezifische Parameter des Patienten über einen vorbestimmten Erfassungszeitraum, Ermitteln eines Eingabewertes für jeden gesundheitsspezifischen Parameter, wobei dieser Eingabewert abhängig ist von einem Wert größer oder gleich dem 0,45-Quantil und kleiner oder gleich dem 0,55 Quantil der über den vorbestimmten Erfassungszeitraum sukzessive für den jeweiligen gesundheitsspezifischen Parameter erfassten Werte, Eingeben der Eingabewerte für die vordefinierten gesundheitsspezifischen Parameter in ein auf einem Computer implementiertes Regressionsmodell oder künstliches neuronales Netzwerk, wobei das Regressionsmodell bzw. das künstliche neuronale Netzwerk auf die Eingabe der Eingabewerte eine Eintrittswahrscheinlichkeit liefert, mit der bei dem Patienten nach einer vorbestimmten Dauer eine Sepsis auftritt.

Gesundheitsspezifische Parameter sind alle messbaren Größen, mit denen der Gesundheitszustand eines Patienten darstellbar ist. Das können Vitalparameter und/oder Laborwerte oder andere messbare, zur Diagnostik verwendbare Werte sein. Der vorbestimmte Erfassungszeitraum ist der Zeitraum, in dem die Werte der gesundheitsspezifischen Parameter zur Ermittlung der Eingabewerte herangezogen werden. Die vorbestimmte Dauer umfasst vorzugsweise einen Zeitraum von zehn Stunden. Das heißt, dass die Eintrittswahrscheinlichkeit einer Sepsis eines Patienten auf die folgenden zehn Stunden gerechnet wird, sodass beispielsweise eine 72 %-Eintrittswahrscheinlichkeit einer Sepsis innerhalb der auf den Ermittlungszeitpunkt folgenden zehn Stunden für den betreffenden Patienten herrscht.

Das Quantil ist ein Lagemaß in der Statistik. Das 0,45-Quantil ist der Wert, für den gilt, dass 45 % aller Werte kleiner oder gleich diesem Wert sind. Das Ermitteln eines Eingabewertes für jeden gesundheitsspezifischen Parameter, wobei dieser Eingabewert abhängig ist von einem Wert größer oder gleich dem 0,45-Quantil und kleiner oder gleich dem 0,55 Quantil der über den vorbestimmten Zeitraum sukzessive für den jeweiligen gesundheitsspezifischen Parameter erfassten Werte, bedeutet, dass ein Wert aus dem Bereich um den Median der Werte herum oder der Median selbst verwendet wird, um den Eingabewert zu bestimmen. In der einfachsten Ausprägung kann der Eingabewert der Median selbst sein, es kann aber auch ein Wert sein, der vom Median abgeleitet ist. Außerdem muss es nicht genau der Median oder ein von dem Median abgeleiteter Wert sein. Vielmehr hat sich im Rahmen der Erfindung herausgestellt, das als Eingabewerte auch solche Werte verwendbar sind, die aus dem Mittenbereich der nach Größe sortierten erfassten Werte stammen, nämlich zwischen dem 0,45-Quantil und dem 0,55-Quantil liegen. Auch hier kann der Eingabewert ein zwischen diesen beiden Quantilen abgeleiteter Wert sein. Vorstellbar ist, dass ein Messgerät eine Fehlfunktion aufweist oder nicht an der vorgesehenen Position misst. Dadurch treten falsche Werte oder Null-Werte innerhalb des vorbestimmten Erfassungszeitraums auf, die bei der Eingabe dieser Parameter in das Modell eine NaN ("keine Zahl") Antwort liefern und somit die Prädiktion erschweren würden. Gerade im Fall unregelmäßiger Messungen oder unregelmäßiger, fehlerhafter Messergebnisse kann erfindungsgemäß gleichwohl eine akkurate Prädiktion erfolgen.

Grundsätzlich kann eine Sepsis anhand verschiedener gesundheitsspezifischer Parameter kategorisiert werden. Gemäß einer bevorzugten Weiterbildung der Erfindung ist jedoch vorgesehen, dass die Eintrittswahrscheinlichkeit der Sepsis mit einem qSOFA-Score $\geq 2$ ermittelt wird. Der qSOFA-Score (quick single organ failure assessment-Score) ist eine reduzierte Form des SOFA-Scores, der ein schnelles und vorteilhaftes Einschätzen eines Patienten hinsichtlich einer Infektion ermöglicht, wobei ein qSOFA $\geq 2$ als Sepsis gilt.

Gemäß einer bevorzugten Weiterbildung der Erfindung ist jedoch vorgesehen, dass der vorbestimmte Zeitraum zwischen einer Stunde und drei Stunden liegt. Vorzugsweise liegt der vorbestimmte Zeitraum zwischen 90 Minuten und 150 Minuten, ganz besonders bevorzugt zwischen 110 und 130 Minuten. Dieser vorbestimmte Zeitraum, in dem die gesundheitsspezifischen Parameter akquiriert werden, gewährleistet gleichzeitig eine frühestmögliche sowie ausreichende Datenakquise für eine möglichst exakte Ermittlung der Eintrittswahrscheinlichkeit einer Sepsis.

Prinzipiell sind verschiedene Kombinationen gesundheitsspezifischer Parameter zur Eingabe in das Regressionsmodell oder das künstliche neuronale Netzwerk möglich. Gemäß einer bevorzugten Weiterbildung der Erfindung ist jedoch vorgesehen, dass die gesundheitsspezifischen Parameter mit nicht-invasiven Messmethoden ermittelt werden. Dadurch ist eine Ermittlung der Eintrittswahrscheinlichkeit einer Sepsis mit Mitteln möglich, die leicht zugänglich sowie schnell verfügbar sind und die keine gewebeschädigenden Folgen aufweisen. Nicht-invasive Messmethoden werden im klinischen Alltag ohnehin routinemäßig in allen Bereichen der stationären Versorgung, aber auch insbesondere im Rahmen der Notfall- und intensivmedizinischen Betreuung und auch in einer ambulanten Betreuung, zur Erhebung von gesundheitsspezifischen Parametern herangezogen.

Grundsätzlich sind verschiedene Kombinationen gesundheitsspezifischer Parameter zur Eingabe in das Regressionsmodell oder das künstliche neuronale Netzwerk möglich.

Gemäß einer bevorzugten Weiterbildung der Erfindung ist jedoch vorgesehen, dass die gesundheitsspezifischen Parameter die Sauerstoffsättigung, die Herzfrequenz, die Atemfrequenz und der systolische Blutdruck des Patienten sind. Diese gesundheitsspezifischen Parameter sind vollständig über die routinemäßige Patientenüberwachung abrufbar und zusätzlich nicht-invasiv. Ebenfalls sind diese Werte zu jedem Zeitpunkt abrufbar, vorausgesetzt ein entsprechendes Messgerät ist aktiv. Dadurch ergeben sich bei zeitkritischen Beurteilungen, wie der Ermittlung der Eintrittswahrscheinlichkeit der Sepsis, Vorteile gegenüber gesundheitsspezifischen Parametern, die möglicherweise ein bildgebendes Verfahren oder eine Untersuchung im Labor benötigen.

Es ist möglich, dass die Eintrittswahrscheinlichkeit der Sepsis zu einem festgelegten Zeitpunkt ermittelt wird. Gemäß einer bevorzugten Weiterbildung der Erfindung ist jedoch vorgesehen, dass die Eintrittswahrscheinlichkeit der Sepsis kontinuierlich ermittelt wird. Dadurch kann eine lückenlose Überwachung des Patienten gewährleistet werden, mithilfe derer flexibel auf sich schnell ändernde Gesundheitslagen reagiert werden kann.

Prinzipiell kann die Eintrittswahrscheinlichkeit der Sepsis auf verschiedene Arten ermittelt werden. Gemäß einer bevorzugten Weiterbildung der Erfindung ist jedoch vorgesehen, dass das Verfahren mit einem künstlichen neuronalen Netzwerk arbeitet, das auf die Erkennung einer Sepsis mit einem qSOFA-Score von mindestens 2 am Menschen trainiert ist. Künstliche neuronale Netzwerke, die entsprechend trainiert sind, liefern insbesondere bei nichtlinearen, hinreichend komplexen Problemen zuverlässige Ergebnisse. Die Korrelationen zwischen gesundheitsspezifischen Parametern sind daher mittels künstlicher neuronaler Netzwerke sehr gut abzubilden.

Grundsätzlich können die Eingabeparameter den Neuronen beliebig kombiniert zugeordnet werden. Gemäß einer bevorzugten Weiterbildung der Erfindung ist jedoch vorgesehen, dass das künstliche neuronale Netzwerk wenigstens vier Neuronen in einer Eingabeschicht aufweist, die je einem der gesundheitsspezifischen Parameter zugeordnet sind. Dadurch sind Korrelationen und Einflüsse einzelner gesundheitsspezifischer Parameter besser zu berücksichtigen und deutlicher abzugrenzen.

Es ist möglich das künstliche neuronale Netzwerk auf verschiedene Arten zu gestalten. Gemäß einer bevorzugten Weiterbildung der Erfindung ist jedoch vorgesehen, dass das trainierte künstliche neuronale Netzwerk mit elastischer Fortpflanzung und mit gewichteter Rückverfolgung ausgebildet ist. Eine elastische Fortpflanzung ist ein iteratives Verfahren zur Bestimmung des Minimums der Fehlerfunktion in einem neuronalen Netz. Jedes Neuron ist mit dem folgenden Neuron einer weiteren Schicht mit einer Kante über eine Gewichtung verbunden. Durch den iterativen Prozess der Minimierung der Fehlerfunktion werden die Wichtungen zwischen den Neuronen angepasst. Die gewichtete Rückverfolgung sieht die Anpassung der für einige oder alle Gewichte mit Hilfe einer Heuristik vor. Algorithmen mit elastischer Fortpflanzung sind äußerst robust in Bezug auf ihre internen Parameter, wodurch, zusammen mit der gewichteten Rückverfolgung ein äußerst robustes Netzwerk trainiert werden kann, dass eine geringere Anfälligkeit gegenüber Rauschen aufweist.

Prinzipiell sind verschiedene Aktivierungsfunktionen in einem künstlichen neuronalen Netzwerk verwendbar. Gemäß einer bevorzugten Weiterbildung der Erfindung ist jedoch vorgesehen, dass ein einzelnes Neuron mit einer der Standardfunktionen, wie z.B. der logistischen Regression oder der Gleichrichter-Funktion, als Aktivierungsfunktion versehen ist. In künstlichen neuronalen Netzen bildet jedes Neuron eine gewichtete Summe seiner Eingänge und leitet den resultierenden skalaren Wert durch eine Funktion, die als Aktivierungsfunktion oder Transferfunktion bezeichnet wird. Wenn die Funktion als lineare Funktion betrachtet wird, führt das Neuron eine lineare Regression oder Klassifizierung durch. Im Allgemeinen wird eine nichtlineare Funktion genutzt, um nichtlineare Regressionen durchzuführen und Klassifizierungsprobleme zu lösen, die nicht linear trennbar sind. Wenn die Funktion eine sigmoidale Funktion ist, die von 0 bis 1 oder -1 bis 1 variiert, kann der Ausgabewert des Neurons als JA/NEIN-Antwort oder binäre Entscheidung mit Hilfe eines Trennpunktes interpretiert werden.

Grundsätzlich kann das künstliche neuronale Netzwerk mit einer Vielzahl an versteckten Schichten ausgestattet sein. Gemäß einer bevorzugten Weiterbildung der Erfindung ist jedoch vorgesehen, dass das künstliche neuronale Netzwerk mit genau einer Eingabeschicht, mit genau einer versteckten Schicht und mit genau einer Ausgabeschicht ausgestaltet ist. Neuronen werden in Schichten zusammengefasst. Verschiedene Schichten können unterschiedliche Transformationen an ihren Eingängen vornehmen. Die Signale wandern von der ersten Schicht (der Eingabeschicht) zur letzten Schicht (der Ausgabeschicht).

Eine von der Eingabeschicht verschiedene Anzahl Neuronen in der versteckten Schicht ist möglich. Gemäß einer bevorzugten Weiterbildung der Erfindung ist jedoch vorgesehen, dass die Anzahl der Neuronen in der versteckten Schicht der Anzahl Neuronen der Eingabeschicht entspricht.

Prinzipiell kann das künstliche neuronale Netzwerk mit einer verschiedenen Anzahl Bias-Neuronen versehen sein. Gemäß einer bevorzugten Weiterbildung der Erfindung ist jedoch vorgesehen, dass das künstliche neuronale Netzwerk mit zwei Bias-Neuronen ausgebildet ist. Das Bias-Neuron hat keine zusätzliche Eingabe innerhalb des neuronalen Netzes und weist immer einen konstanten Wert auf, wodurch es als neutrale Instanz in Kombination mit der Gewichtung die Ergebnisse im künstlichen Neuronalen Netz in eine vorgegebene Richtung verschieben kann.

Erfindungsgemäß ist weiter ein Vorhersagesystem zur Berechnung der Eintrittswahrscheinlichkeit einer Sepsis eines Patienten vorgesehen, wobei das Vorhersagesystem eine Datenbank, eine Analyseeinheit und einen Computer aufweist, wobei die sukzessiv erfassten Werte für die vier vordefinierten gesundheitsspezifischen Parameter des Patienten über den vorbestimmten Erfassungszeitraum in der Datenbank speicherbar sind und der Eingabewert für jeden gesundheitsspezifischen Parameter der größer oder gleich dem 0,45-Quantil und kleiner oder gleich dem 0,55-Quantil der über den vorbestimmten Zeitraum sukzessive für den jeweiligen gesundheitsspezifischen Parameter erfassten Werte mit der Analyseeinheit ermittelbar ist, wobei die Eingabewerte für die vordefinierten gesundheitsspezifischen Parameter in das auf einem Computer implementierte Regressionsmodell oder das künstliche neuronales Netzwerk eingebbar sind, wobei eine Eintrittswahrscheinlichkeit, mit der bei dem Patienten nach einer vorbestimmten Dauer eine Sepsis auftritt, mit dem auf dem Computer implementierten Regressionsmodell bzw. dem künstlichen neuronalen Netzwerk auf die Eingabe der Eingabewerte lieferbar ist.

Die Datenbank weist vorzugsweise eine Schnittstelle zu einem Softwaresystem mit Patientendaten auf und bezieht diese aus derselben. Die Daten sind dabei aus Systemen, wie dem Krankenhausinformationssystem (KIS), dem Patientendatenmanagementsystem (PDMS), einem System zur ambulanten Patientendokumentation oder einem Softwaresystem eines mobil tragbaren Medizin- oder Lifestyleprodukt, beziehbar und werden in einer speziellen Struktur abgespeichert, sodass sie von der Analyseeinheit genutzt werden können. Die Datenbank kann als das Software-Framework fungieren, das es ermöglicht, den Zugriff auf den Datensatz anzuzeigen, zu erstellen, zu verwalten und zu kontrollieren. Die Datenbank kann es ermöglichen, erforderliche Daten im Datensatz zu erstellen, zu lesen, zu aktualisieren und zu löschen. Sie kann wie eine Schnittstelle zwischen den Programmen und Daten funktionieren. Dazu sind die Daten in der Datenbank konsistent und genau. Dies bedeutet, dass die Daten in allen Datenbanken für alle Benutzer konsistent und korrekt sind. Durch das Datenbankverwaltungssystems besteht Zugriff auf gut verwaltete und synchronisierte Daten. Weiter ist Datenkonsistenz in der Datenbank gewährleistet; es gibt keine Datenredundanz. Außerdem werden alle Datenbankänderungen vorzugsweise sofort widergespiegelt. Das Datenbankverwaltungssystem hilft, schnelle Antworten auf Abfragen zu geben, wodurch der Datenzugriff genau und schneller erfolgt. Die Analyseeinheit kann in dem Computer ausgebildet sein, der das Regressionsmodell oder das künstliche neuronale Netzwerk enthält.

Prinzipiell können die gesundheitsspezifischen Parameter zu festgelegten Zeitpunkten übermittelt werden. Gemäß einer bevorzugten Weiterbildung der Erfindung ist jedoch vorgesehen, dass die gesundheitsspezifischen Parameter der Patienten in der Datenbank über eine Schnittstelle mit dem medizinischen Dokumentationssystem verbunden sind, wobei die gesundheitsspezifischen Parameter kontinuierlich übermittelbar sind. Dadurch wird eine kontinuierliche Berechnung der Eintrittswahrscheinlichkeit ermöglicht, mit der bei dem Patienten nach einer vorbestimmten Dauer eine Sepsis auftritt.

Gemäß einer bevorzugten Weiterbildung der Erfindung ist vorgesehen, dass das Vorhersagesystem eine Anzeigeeinheit aufweist und der vorbestimmte Erfassungszeitraum des Patienten über die mit einer Benutzeroberfläche versehene Anzeigeeinheit steuerbar ist. Dadurch kann flexibel auf die gemessenen Parameter und die Eintrittswahrscheinlichkeit, mit der bei dem Patienten nach einer vorbestimmten Dauer eine Sepsis auftritt, reagiert werden. Bei niedrigem Risiko können gegebenenfalls Ressourcen geschont werden durch kürzere vorbestimmte Erfassungszeiträume. Bei höherem Risiko hingegen könnte eine genauere Überwachung erzielt werden durch längere vorbestimmte Erfassungszeiträume. Die Anzeigeeinheit kann in dem Computer ausgebildet sein, der das Regressionsmodell oder das künstliche neuronale Netzwerk enthält.

Gemäß einer bevorzugten Weiterbildung der Erfindung ist vorgesehen, dass die Eintrittswahrscheinlichkeit mit der bei dem Patienten nach einer vorbestimmten Dauer eine Sepsis auftritt in der Benutzeroberfläche kontinuierlich einsehbar ist.

Es ist möglich, dass ausschließlich die aktuelle Eintrittswahrscheinlichkeit mit der bei dem Patienten nach einer vorbestimmten Dauer eine Sepsis auftritt abrufbar ist. Gemäß einer bevorzugten Weiterbildung der Erfindung ist jedoch vorgesehen, dass die Eintrittswahrscheinlichkeit mit der bei dem Patienten nach einer vorbestimmten Dauer eine Sepsis auftritt als zeitlicher Verlauf für den betrachteten Patienten abrufbar ist.

In der Zeichnung zeigen

Fig. 1    schematisch ein Vorhersagesystem zur Berechnung der Eintrittswahrscheinlichkeit einer Sepsis eines Patienten gemäß einem bevorzugten Ausführungsbeispiel der Erfindung,

Fig. 2    schematisch ein künstliches neuronales Netzwerk gemäß einem bevorzugten Ausführungsbeispiel der Erfindung und

Fig. 3    schematisch eine Benutzeroberfläche gemäß einem bevorzugten Ausführungsbeispiel der Erfindung.

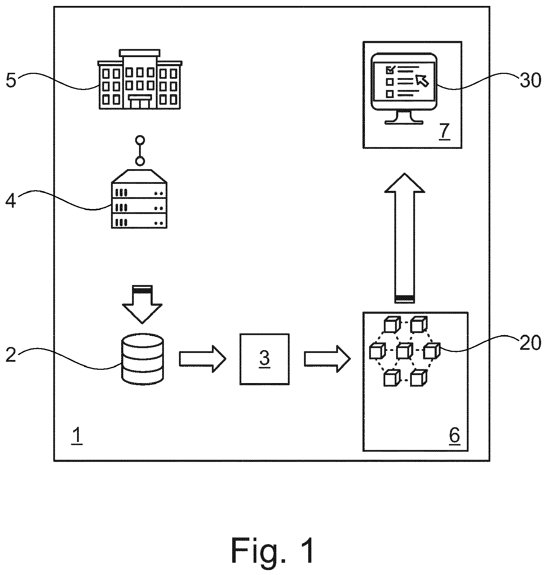

Aus Fig. 1 ist schematisch ein Vorhersagesystem 1 zur Ermittlung einer Eintrittswahrscheinlichkeit einer Sepsis eines Patienten 37 mit einer Datenbank 2, mit einer Analyseeinheit 3 und mit einem Computer 6 ersichtlich. Ein Patient 37 befindet sich auf einer Intensiveinheit eines Krankenhauses 5 und ist verbunden mit einem Gerät zur Atemunterstützung, einer Blutdruckmanschette sowie einem Pulsoximeter, wodurch sich wiederum vier verschiedene nicht-invasive gesundheitsspezifische Parameter als Werte gewinnen lassen: die Herzfrequenz 33, die Atemfrequenz 34, die Sauerstoffsättigung 35 und der systolische Blutdruck 36. Diese gesundheitsspezifischen Parameter werden kontinuierlich gemessen und die Werte werden automatisiert in einem Krankenhaussystem 4 gespeichert, welches über eine Schnittstelle mit einer Datenbank 2 verbunden ist.

Die Datenbank 2 bezieht die gemessenen Werte kontinuierlich und formatiert diese derart, dass der Eingabewert für jeden gesundheitsspezifischen Parameter mit der Analyseeinheit 3 ermittelbar ist. Diese greift ebenfalls kontinuierlich auf die in der Datenbank 2 enthaltenen gesundheitsspezifischen Parameter zu. Für einen vorbestimmten Erfassungszeitraum 32, der variabel in der Benutzeroberfläche 30 der Anzeigeeinheit 7 steuerbar ist, werden alle Werte für jeden gesundheitsspezifischen Parameter für den Patienten in die Analyseneinheit 3 geladen. Die Eingabewerte für den Computer 6 und das darin enthaltene künstliche neuronale Netzwerk 20 werden für jeden gesundheitsspezifischen Parameter mittels der Analyseeinheit 3 ermittelt, wobei dieser Eingabewert abhängig ist von einem Wert größer oder gleich dem 0,45-Quantil und kleiner oder gleich dem 0,55 Quantil der über den vorbestimmten Erfassungszeitraum 32 sukzessive für den jeweiligen gesundheitsspezifischen Parameter erfassten Werte.

Die mit dem künstlichen neuronalen Netzwerk 20 in dem Computer 6 ermittelte Eintrittswahrscheinlichkeit mit der bei dem Patienten nach einer vorbestimmten Dauer eine Sepsis auftritt 28 wird mittels der über die Anzeigeeinheit 7 wiedergegebenen Benutzeroberfläche 30 geliefert.

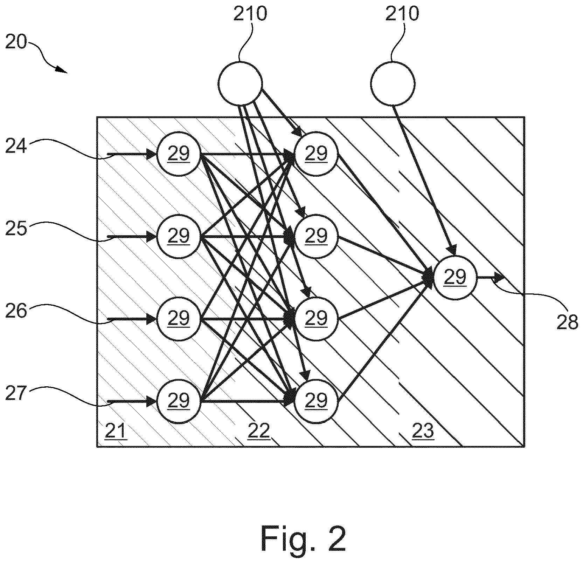

Das in Fig. 2 abgebildete künstliche neuronale Netzwerk 20 weist eine Eingabeschicht 21, eine versteckte Schicht 22 sowie eine Ausgabeschicht 23 auf. Die mit der Analyseeinheit 3 ermittelten Eingabewerte für die Herzfrequenz 24, die Atemfrequenz 25, die Sauerstoffsättigung 26 und den systolischen Blutdruck 27 werden innerhalb der Eingabeschicht 21 an das künstliche neuronale Netzwerk 20 übergeben. Jedes Neuron 29 überträgt eine Eingabe, beispielsweise $p$ Merkmale $(x_{1i},...,x_{pi})$, die einen Patienten $i$ beschreiben in eine Ausgabe $y(x)$, wobei

$$y(x) = f\left(\beta_0 + \sum_{l=1}^{p} \beta_l x_l\right).$$

Die Wahrscheinlichkeit der Zugehörigkeit zu einer Klasse ermittelt wird, indem die eingehenden Daten mit bestimmten Wichtungen $\beta_l$, $l = 0, 1, ..., p$ multipliziert und die Aktivierungsfunktion $f$ angewandt und summiert wird.

Jedes Neuron 29 der Eingabeschicht 21 ist mit jedem Neuron 29 der versteckten Schicht 22 über eine Wichtung mit einer Kante verbunden. Die Anzahl der Neuronen 29 der versteckten Schicht 22 des künstlichen neuronalen Netzwerks 20 in Fig.2 stimmt mit der Anzahl Neuronen 29 der Eingabeschicht 21 überein. Die Ausgabeschicht 23 weist ein einzelnes Neuron 29 auf. Jedes Neuron 29 der versteckten Schicht 22 ist mit dem einzelnen Neuron 29 der Ausgabeschicht 23 über eine Wichtung mit einer Kante verbunden. Zusätzlich sind in dem künstlichen neuronalen Netzwerk 20 zwei Bias-Neuronen 210 enthalten. Das erste Bias-Neuron 210 ist zwischen der Eingabeschicht 21 und der versteckten Schicht 22 lokalisiert und mit jedem Neuron 29 der versteckten Schicht 22 über eine Wichtung mit einer Kante verbunden. Das zweite Bias-Neuron 210 ist zwischen der versteckten Schicht 22 und der Ausgabeschicht 23 lokalisiert und mit dem Neuron 29 der Ausgabeschicht 23 über eine Wichtung mit einer Kante verbunden. Das Neuron 29 der Ausgabeschicht 23 liefert die Eintrittswahrscheinlichkeit mit der bei dem Patienten nach einer vorbestimmten Dauer eine Sepsis auftritt 28. Dazu ist die Sepsis klassifiziert über einen qSOFA-Score von mindestens 2.

Die Leistungsfähigkeit des künstlichen neuronalen Netzes 20 wird über einen diagnostischen Test ermittelt. Er dient der Beurteilung der Diagnosegenauigkeit bzw. Prädiktionsgenauigkeit. Der Befund bezüglich einer Sepsis kann positiv oder negativ sein. Hinzu kommt, dass dieser Befund nicht immer zutreffend ist. Somit muss man weiter nach richtig positiv (RP), richtig negativ (RN), falsch positiv (FP) und falsch negativ (FN) differenzieren. Die zugehörigen Häufigkeiten lassen sich über die nachfolgende Tabelle darstellen.

Tabelle 1: Vierfeldertafel zum diagnostischen Test.

| Befund | Test | | Summe |
|---|---|---|---|
| | positiv | negativ | |
| vorhanden | $a = RP$ | $b = FN$ | $a + b$ [Betroffene] |
| fehlend | $c = FP$ | $d = RN$ | $c + d$ [nicht Betroffene] |
| Summe | $a + c$ [Testpositive] | $b + d$ [Testnegative] | $a + b + c + d = n$ |

Allgemeiner und analog zu Tabelle 1 lässt sich dies mit den entsprechenden mathematischen Bezeichnungen darstellen:

Tabelle 2: Allgemeine Vierfeldertafel zum diagnostischen Test.

| | $T^+$ | $T^-$ | $\Sigma$ |
|---|---|---|---|
| $K$ | $a$ | $b$ | $a + b$ |
| $\overline{K}$ | $c$ | $d$ | $c + d$ |
| $\Sigma$ | $a + c$ | $b + d$ | $n$ |

Wichtige Kenngrößen zum diagnostischen Test sind hierbei

1. der Anteil negativ Getesteter unter den nicht Betroffenen,
2. der Anteil positiv Getesteter unter den Betroffenen,
3. der Anteil nicht Betroffener unter den negativ Getesteten und
4. der Anteil Betroffener unter den positiv Getesteten.

Bezeichnet werden die genannten vier Größen mit:

1. *Spezifität*:

$$P(T^- | \overline{K}) = \frac{a}{a+b},$$

2. *Sensitivität*:

$$P(T^+|K) = \frac{d}{c+d},$$

3. **Negativer Voraussagewert** (*NPV*):

$$P(\overline{K}|T^-) = \frac{d}{b+d}$$

und

4. **Positiver Voraussagewert** (*PPV*):

$$P(K|T^+) = \frac{a}{a+c}.$$

Weitere wichtige Größen zum diagnostischen Test sind die Prävalenz und die Wahrscheinlichkeitsquotienten. Die Prävalenz gibt den Anteil Erkrankter in der Stichprobe an. Der positive Wahrscheinlichkeitsquotient gibt die Wahrscheinlichkeit, an ein positives Testergebnis bei Betroffenen zu finden, im Verhältnis zur Wahrscheinlichkeit, ein positives Testergebnis bei nicht Betroffenen zu finden (*LR*+). Analog kann *LR*- für ein negatives Testergebnis interpretiert werden:

$$LR^+ = \frac{Sensitivität}{1-Spezifität},$$

$$LR^+ = \frac{1-Sensitivität}{Spezifität}.$$

*LR*+ und *LR*- haben den Vorteil, dass sie unabhängig von der Prävalenz sind und *Sensitivität* und *Spezifität* einbeziehen.

Da die Messgröße zur Prädiktion des Befundes metrisch ist, ist die Bestimmung eines Grenzwertes notwendig. Dieser Grenzwert wird erhalten, indem *Sensitivität* und 1 - *Spezifität* gegeneinander grafisch aufgetragen werden. Der ideale Grenzwert ergibt sich durch Parallelverschiebung der Winkelhalbierenden an dem äußersten Berührungspunkt der Kurve. An dieser Stelle liegt der ideale Grenzwert *c* , für den die Summe aus *Sensitivität* und *Spezifität* maximal ist. Diese maximale Summe wird als Youden-Index mit

$$J_c = \max\left(Sensitivität(c) + Spezifität(c) - 1\right)$$

bezeichnet.

Die Leistungsfähigkeit für die Lieferung der Eintrittswahrscheinlichkeit mit der bei dem Patienten nach einer vorbestimmten Dauer eine Sepsis auftritt 28 ist in Tabelle 3 dargestellt.

Tabelle 3: Prädiktionsgenauigkeit.

| Index | Value |
|---|---|
| PPV | 0,69 |
| NPV | 0,839 |
| Sensitivität | 0,853 |
| Spezifität | 0,667 |
| Grenzwert | -0,461 |

(fortgesetzt)

| Index | Value |
|---|---|
| RP | 29 |
| RN | 26 |
| FP | 13 |
| FN | 5 |
| AUC (95%-CI) | 0,814(0,717 - 0,912) |
| LR+ | 2,559 |
| LR- | 0,221 |
| Youden-Index | 0,52 |

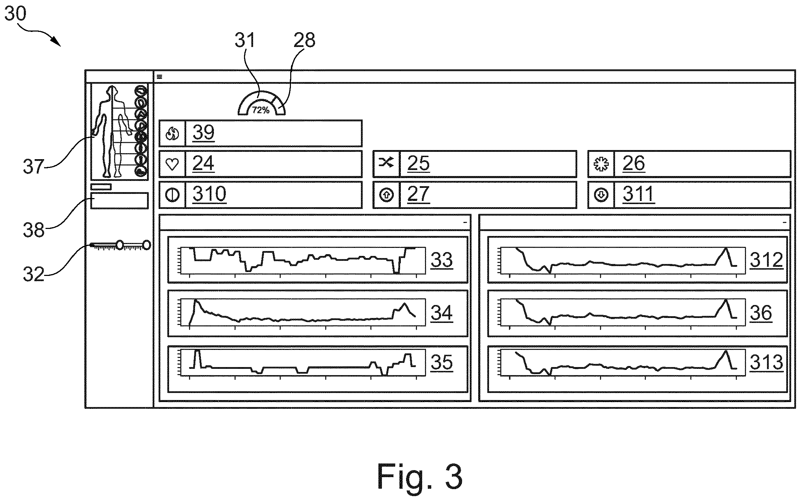

In Fig. 3 ist die Benutzeroberfläche 30 dargestellt, die die Eingabewerte der gesundheitsspezifischen Parameter: die Herzfrequenz 24, die Atemfrequenz 25, die Sauerstoffsättigung 26 und den systolischen Blutdruck 27 anzeigt. Weiter werden in der Benutzeroberfläche die kontinuierlich erfassten Werte der gesundheitsspezifischen Parameter, die Herzfrequenz 33, die Atemfrequenz 34, die Sauerstoffsättigung 35 und der systolische Blutdruck 36 gegen die Zeit des vorbestimmten Erfassungszeitraums 32 aufgetragen. Diese Daten sind für jeden Patienten 37, für den sie erhoben werden, über eine Eingabe in einem Auswahlbereich 38 abrufbar, indem der betrachtete Patient 37 über den Auswahlbereich 38 gewechselt wird. Zu jedem Patienten 37 wird die Eintrittswahrscheinlichkeit geliefert, mit der bei dem Patienten 37 nach einer vorbestimmten Dauer eine Sepsis auftritt 28. Diese Eintrittswahrscheinlichkeit wird als ein Tachometer 31 sowie als eine Ausgabe 39 visualisiert. Die Ausgabe 39 zeigt zusätzlich den vorbestimmten Erfassungszeitraum 32 an. Weitere Eingabewerte wichtiger Parameter, wie einen mittleren arteriellen Druck 310 und einen diastolischen Druck 311 sowie die zugehörigen Werte 312 und 313 sind ebenfalls anzeigbar.

Bezugszeichenliste

| 1 | Vorhersagesystem |
| 2 | Datenbank |
| 3 | Analyseeinheit |
| 4 | Krankenhaussystem |
| 5 | Krankenhaus |
| 6 | Computer |
| 7 | Anzeigeeinheit |
| 20 | Künstliches neuronales Netzwerk |
| 21 | Eingabeschicht |
| 22 | Versteckte Schicht |
| 23 | Ausgabeschicht |
| 24 | Eingabewert Herzfrequenz |
| 25 | Eingabewert Atemfrequenz |
| 26 | Eingabewert Sauerstoffsättigung |
| 27 | Eingabewert systolischer Blutdruck |
| 28 | Eintrittswahrscheinlichkeit mit der bei dem Patienten nach einer vorbestimmten Dauer eine Sepsis auftritt |
| 29 | Neuron |
| 210 | Bias-Neuron |
| 30 | Benutzeroberfläche |
| 31 | Tachometer |
| 32 | vorbestimmter Erfassungszeitraum |
| 33 | Werte Herzfrequenzen |
| 34 | Werte Atemfrequenzen |
| 35 | Werte Sauerstoffsättigungen |
| 36 | Werte systolische Blutdrücke |
| 37 | Patient |

| 38 | Auswahlbereich |
|---|---|
| 39 | Ausgabe |
| 310 | Eingabewert mittlerer arterieller Druck |
| 311 | Eingabewert diastolischer Druck |
| 312 | Werte mittlere arterielle Drücke |
| 313 | Werte diastolische Drücke |

**Patentansprüche**

1. Verfahren zur Ermittlung der Eintrittswahrscheinlichkeit einer Sepsis eines Patienten mit folgenden Verfahrensschritten:

   sukzessives Erfassen von jeweiligen Werten (33, 34, 35, 36) für wenigstens vier vordefinierte gesundheitsspezifische Parameter des Patienten (37) über einen vorbestimmten Erfassungszeitraum (32),
   Ermitteln eines Eingabewertes (24, 25, 26, 27) für jeden gesundheitsspezifischen Parameter, wobei dieser Eingabewert (24, 25, 26, 27) abhängig ist von einem Wert größer oder gleich dem 0,45-Quantil und kleiner oder gleich dem 0,55 Quantil der über den vorbestimmten Erfassungszeitraum (32) sukzessive für den jeweiligen gesundheitsspezifischen Parameter erfassten Werte (33, 34, 35, 36),
   Eingeben der Eingabewerte (24, 25, 26, 27) für die vordefinierten gesundheitsspezifischen Parameter in ein auf einem Computer (6) implementiertes Regressionsmodell oder künstliches neuronales Netzwerk (20), wobei das Regressionsmodell bzw. das künstliche neuronale Netzwerk (20) auf die Eingabe der Eingabewerte (24, 25, 26, 27) eine Eintrittswahrscheinlichkeit liefert, mit der bei dem Patienten nach einer vorbestimmten Dauer eine Sepsis auftritt (28).

2. Verfahren zur Ermittlung der Eintrittswahrscheinlichkeit einer Sepsis eines Patienten nach dem vorigen Anspruch, wobei die Eintrittswahrscheinlichkeit der Sepsis mit einem qSOFA-Score $\geq 2$ ermittelt wird.

3. Verfahren zur Ermittlung der Eintrittswahrscheinlichkeit einer Sepsis eines Patienten nach einem der vorigen Ansprüche, wobei der vorbestimmte Erfassungszeitraum (32) zwischen einer Stunde und drei Stunden liegt.

4. Verfahren zur Ermittlung der Eintrittswahrscheinlichkeit einer Sepsis eines Patienten nach einem der vorigen Ansprüche, wobei die gesundheitsspezifischen Parameter mit nicht-invasiven Messmethoden ermittelt werden.

5. Verfahren zur Ermittlung der Eintrittswahrscheinlichkeit einer Sepsis eines Patienten nach einem der vorigen Ansprüche, wobei die gesundheitsspezifischen Parameter die Sauerstoffsättigung, die Herzfrequenz, die Atemfrequenz und der systolische Blutdruck des Patienten sind.

6. Verfahren zur Ermittlung der Eintrittswahrscheinlichkeit einer Sepsis eines Patienten nach einem der vorigen Ansprüche, wobei die Eintrittswahrscheinlichkeit der Sepsis kontinuierlich ermittelt wird.

7. Verfahren zur Ermittlung der Eintrittswahrscheinlichkeit einer Sepsis eines Patienten nach einem der vorigen Ansprüche, wobei das Verfahren mit einem künstlichen neuronalen Netzwerk (20) arbeitet, das auf die Erkennung einer Sepsis mit einem qSOFA-Score von mindestens 2 am Menschen trainiert ist.

8. Verfahren zur Ermittlung der Eintrittswahrscheinlichkeit einer Sepsis eines Patienten nach Anspruch 7, wobei das künstliche neuronale Netzwerk wenigstens vier Neuronen (29) in einer Eingabeschicht (21) aufweist, die je einem der gesundheitsspezifischen Parameter zugeordnet sind.

9. Verfahren zur Ermittlung der Eintrittswahrscheinlichkeit einer Sepsis eines Patienten nach Anspruch 7 oder 8, wobei das trainierte künstliche neuronale Netzwerk (20) mit elastischer Fortpflanzung und mit gewichteter Rückverfolgung ausgebildet ist.

10. Verfahren zur Ermittlung der Eintrittswahrscheinlichkeit einer Sepsis eines Patienten nach einem der Ansprüche 7 bis 9, wobei das künstliche neuronale Netzwerk (20) mit einer Eingabeschicht (21), mit einer versteckten Schicht (22) und einer Ausgabeschicht (23) ausgestaltet ist.

11. Verfahren zur Ermittlung der Eintrittswahrscheinlichkeit einer Sepsis eines Patienten nach Anspruch 10, wobei die Anzahl der Neuronen (29) in der versteckten Schicht (22) der Anzahl Neuronen (29) der Eingabeschicht (21) entspricht.

12. Vorhersagesystem (1) zur Berechnung der Eintrittswahrscheinlichkeit einer Sepsis eines Patienten (37), wobei das Vorhersagesystem (1) eine Datenbank (2), eine Analyseeinheit (3) und einen Computer (6) aufweist, wobei

sukzessive erfassten Werte (33, 34, 35, 36) für wenigstens vier vordefinierte gesundheitsspezifische Parameter eines Patienten (37) über einen vorbestimmten Erfassungszeitraum in der Datenbank (2) speicherbar sind und ein Eingabewert (24, 25, 26, 27) für jeden gesundheitsspezifischen Parameter der größer oder gleich dem 0,45-Quantil und kleiner oder gleich dem 0,55-Quantil der über einen vorbestimmten Erfassungszeitraum (32) sukzessive für den jeweiligen gesundheitsspezifischen Parameter erfassten Werte (33, 34, 35, 36) mit der Analyseeinheit (3) ermittelbar ist, wobei
die Eingabewerte (24, 25, 26, 27) für die vordefinierten gesundheitsspezifischen Parameter in ein auf dem Computer (6) implementiertes Regressionsmodell oder ein künstliches neuronales Netzwerk (20) eingebbar sind,
wobei eine Eintrittswahrscheinlichkeit, mit der bei dem Patienten nach einer vorbestimmten Dauer eine Sepsis auftritt (28), mit dem auf dem Computer (6) implementierten Regressionsmodell bzw. dem künstlichen neuronalen Netzwerk (20) auf die Eingabe der Eingabewerte hin lieferbar ist.

13. Vorhersagesystem (1) zur Berechnung der Eintrittswahrscheinlichkeit einer Sepsis eines Patienten (37) nach Anspruch 12, wobei die Berechnung der Eintrittswahrscheinlichkeit mit der bei dem Patienten nach einer vorbestimmten Dauer eine Sepsis auftritt (28) mit einem mit einer GPU-Architektur versehenen Computer (6) durchführbar ist.

14. Vorhersagesystem (1) zur Berechnung der Eintrittswahrscheinlichkeit einer Sepsis eines Patienten (37) nach Anspruch 12 oder 13, wobei die gesundheitsspezifischen Parameter der Patienten (37) in der Datenbank über eine Schnittstelle mit dem Krankenhaussystem (4) verbunden sind, wobei die gesundheitsspezifischen Parameter kontinuierlich übermittelbar sind.

15. Vorhersagesystem (1) zur Berechnung der Eintrittswahrscheinlichkeit einer Sepsis eines Patienten (37) nach einem der Ansprüch 12 bis 14, wobei das Vorhersagesystem (1) zusätzlich eine Anzeigeeinheit (7) aufweist und der vorbestimmte Erfassungszeitraum (32) des Patienten (37) über die mit einer Benutzeroberfläche (30) versehene Anzeigeeinheit (7) steuerbar ist.

Fig. 1

Fig. 2

Fig. 3

Europäisches Patentamt
European Patent Office
Office européen des brevets

**EUROPÄISCHER RECHERCHENBERICHT**

Nummer der Anmeldung

EP 23 17 6088

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2021/059597 A1 (CHUNG CHIEW YUAN [SG] ET AL) 4. März 2021 (2021-03-04) * Absätze 41, 63, 61, 43, 68, 5, 42, 19 * ----- | 1-15 | INV. G16H50/20 G16H50/30 |
| A | SIMON MEYER LAURITSEN ET AL: "Explainable artificial intelligence model to predict acute critical illness from electronic health records", ARXIV.ORG, CORNELL UNIVERSITY LIBRARY, 201 OLIN LIBRARY CORNELL UNIVERSITY ITHACA, NY 14853, 3. Dezember 2019 (2019-12-03), XP081544112, * Seiten 8-9 * ----- | 1-15 | |

**RECHERCHIERTE SACHGEBIETE (IPC)**

G16H

Der vorliegende Recherchenbericht wurde für alle Patentansprüche erstellt

| Recherchenort | Abschlußdatum der Recherche | Prüfer |
|---|---|---|
| München | 16. Oktober 2023 | Wittke, Claudia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 17 6088

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-10-2023

| Im Recherchenbericht angeführtes Patentdokument | Datum der Veröffentlichung | Mitglied(er) der Patentfamilie | Datum der Veröffentlichung |
|---|---|---|---|
| US 2021059597 A1 | 04-03-2021 | CN 112447289 A | 05-03-2021 |
| | | EP 3785611 A1 | 03-03-2021 |
| | | US 2021059597 A1 | 04-03-2021 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2017165693 A1
- DE 102020214050 A1

- WO 2006061644 A1